# EUROPEAN PATENT APPLICATION

(11) **EP 4 553 389 A1**
(43) Date of publication of application: **14.05.2025**
(21) Application number: 23215562.2
(22) Date of filing: 11.12.2023
(51) Int. Cl.: F24C 15/20

(54) **AIR POLLUTION PREVENTION SYSTEM FOR KITCHEN UNIT IN INDOOR FIELD**

(30) Priority: 10.11.2023 TW 112143508
(71) Applicant: Microjet Technology Co., Ltd., Hsinchu (TW)
(72) Inventor: Mou, Hao-Jan, Hsinchu (TW); Wu, Chin-Chuan, Hsinchu (TW); Huang, Chi-Feng, Hsinchu (TW)
(74) Representative: Uexküll & Stolberg

(57) **Abstract**

An air pollution prevention system for a kitchen unit in an indoor field includes a cooking device (H), negative pressure exhausting devices (A), gas detectors (1) and a cloud computing server (2). The negative pressure exhausting devices (A) are disposed above and in front of the cooking device (H) and respectively connected to exhausting channels (B). Each of the negative pressure exhausting devices (A) includes a fan (A1) and a filter element (A2). The gas detectors (1) detect air pollution and output air pollution information. The cloud computing server (2) receives the air pollution information, calculates the concentration of air pollution, and issues the control command to the negative pressure exhausting devices (A) to enable the fans (A1), so as to guide the air pollution to flow into exhausting channels (B) and pass through the filter elements (A2) for exhausting to the outdoor, thereby achieving a gas state in the kitchen unit with a level of air pollution close to zero.

## Description

### FIELD OF THE INVENTION

The present disclosure relates to an air pollution prevention system in an indoor field, and more particularly to an air pollution prevention system for a kitchen unit in the indoor field.

### BACKGROUND OF THE INVENTION

Suspended particles are solid particles or droplets contained in the air. Due to their extremely fine size, the suspended particles may enter the lungs of human body through the nasal hairs in the nasal cavity easily, causing inflammation in the lungs, asthma or cardiovascular disease. If other pollutant compounds are attached to the suspended particles, it will further increase the harm to the respiratory system. In recent years, the problem of air pollution is getting worse. In particular, the concentration of particle matters (e.g., PM_{2.5}) is often reaching excessively high levels. Therefore, the monitoring to the concentration of the gas suspended particles is taken more and more seriously. However, the gas flows unstably due to variable wind direction and air volume, and the general gas-quality monitoring station is located in a fixed place. Under this circumstance, it is impossible for people to check the concentration of suspended particles in current environment.

Furthermore, in recent years, modern people are placing increasing importance on the quality of the air in their surroundings. For example, carbon monoxide, carbon dioxide, volatile organic compounds (VOC), PM2.5, nitric oxide, sulfur monoxide and even the suspended particles contained in the air are exposed in the environment to affect the human health, and even endanger the life seriously. Therefore, the quality of environmental air has attracted the attention of various countries. At present, how to detect the air quality and avoid the harm is a crucial issue that urgently needs to be solved.

In order to confirm the quality of the air, it is feasible to use a gas sensor to detect the air in the surrounding environment. If the detection information can be provided in real time to warn people in the environment, it is helpful of avoiding the harm and facilitates people to escape the hazard immediately, preventing the hazardous gas exposed in the environment from affecting the human health and causing the harm. Therefore, it is considered a valuable application to use a gas sensor to detect the air in the surrounding environment.

In addition, it is not easy to control the indoor air quality. Besides the outdoor air quality, indoor air-conditioning conditions and pollution sources are the major factors affecting the indoor air quality. It is necessary to intelligently and quickly detect indoor air pollution sources in various indoor fields, effectively remove the indoor air pollution to form a clean and safe breathing gas state, and monitor the indoor air quality in real time anytime, anywhere. Therefore, it is a main subject developed in the present disclosure to provide an air pollution prevention system in the indoor field, especially an air pollution prevention system for the kitchen unit in the indoor field, for directly exhausting the air pollution, so as to prevent the cook from smelling fumes and avoid the air pollution from diffusing to other spaces.

### SUMMARY OF THE INVENTION

The major object of the present disclosure is to provide an air pollution prevention system for a kitchen unit in an indoor field. Due to the occurrence and mobility of indoor air pollution from the kitchen unit in the indoor field at any moment, in the present disclosure, a plurality of negative pressure exhausting devices are disposed above and in front of the cooking device, and a plurality of gas detectors are disposed on the plurality of negative pressure exhausting devices for detecting the air pollution and outputting air pollution information and also for receiving a control command to enable the plurality of negative pressure exhausting devices. Moreover, a cloud computing server is employed to receive the air pollution information detected by the plurality of gas detectors, store the air pollution information to an air pollution database, perform an intelligence computing to determine the concentration of air pollution, and issue the control command to the plurality of negative pressure exhausting devices for enabling fans thereof and also adjusting the air volume and operation time of the fans, so that the air pollution from the kitchen unit is rapidly guided to pass through a filter element for filtration and exhaust to the outdoor. Whereby, the air pollution is directly drawn out for preventing the cook from smelling fumes, and avoiding the diffusion of air pollution to other spaces, such as the living room.

For achieving the object above, the present disclosure provides an air pollution prevention system for a kitchen unit in an indoor field. The air pollution prevention system includes a cooking device disposed in a kitchen unit in an indoor field, wherein an air pollution is generated when the cooking device is enabled to cook; a plurality of negative pressure exhausting devices disposed above and in front of the cooking device and respectively connected to an exhausting channel, wherein each of the plurality of negative pressure exhausting devices includes at least one fan and at least one filter element, and the at least one fan is controlled to generate a negative pressure for guiding the air pollution to flow into the respective exhausting channel and pass through the at least one filter element for filtration and exhausting to an outdoor; a plurality of gas detectors disposed on the plurality of negative pressure exhausting devices for detecting the air pollution and outputting air pollution information and for receiving a control command to enable the plurality of negative pressure exhausting devices; and a cloud computing server receiving the air pollution information detected by the plurality of negative pressure exhausting devices, storing the air pollution information to an air pollution database, performing an intelligent computing for determining a concentration of the air pollution, and issuing the control command to the plurality of negative pressure exhausting devices to enable the fans of the plurality of negative pressure exhausting devices, thereby rapidly guiding the air pollution from the kitchen unit to pass through the filter elements for filtration and purification and to exhaust to the outdoor. In that, a transmission of the control command is enabled when the cooking device is enabled to cook, and after the plurality of gas detectors receive the control command, the plurality of negative pressure exhausting devices are enabled, so that the air pollution from the kitchen unit is guided to rapidly flow into the plurality of negative pressure exhausting devices for exhausting to the outdoor, thereby achieving a gas state in the kitchen unit with a level of air pollution close to zero.

### BRIEF DESCRIPTION OF THE DRAWINGS

The above contents of the present disclosure will become more readily apparent to those ordinarily skilled in the art after reviewing the following detailed description and accompanying drawings, in which:
FIG. 1 is a schematic view illustrating an air pollution prevention system for a kitchen unit in an indoor field according to an embodiment of the present disclosure;
FIG. 2 is a schematic view illustrating a combined filter element disposed in an exhausting channel of the air pollution prevention system for the kitchen unit in the indoor field according to the embodiment of the present disclosure;
FIG. 3 is a schematic perspective view illustrating a gas detector according to an embodiment of the present disclosure;
FIG. 4A is a schematic perspective view (1) illustrating a gas detection main part according to the embodiment of the present disclosure;
FIG. 4B is a schematic perspective view (2) illustrating the gas detection main part according to the embodiment of the present disclosure;
FIG. 4C is an exploded view illustrating the gas detection main part according to the embodiment of the present disclosure;
FIG. 5A is a schematic perspective view (1) illustrating a base according to the embodiment of the present disclosure;
FIG. 5B is a schematic perspective view (2) illustrating the base according to the embodiment of the present disclosure;
FIG. 6 is a schematic view (3) illustrating the base according to the embodiment of the present disclosure;
FIG. 7A is a schematic exploded view illustrating the combination of a piezoelectric actuator and the base according to the embodiment of the present disclosure;
FIG. 7B is a schematic perspective view illustrating the combination of the piezoelectric actuator and the base according to the embodiment of the present disclosure;
FIG. 8A is a schematic exploded view (1) illustrating the piezoelectric actuator according to the embodiment of the present disclosure;
FIG. 8B is a schematic exploded view (2) illustrating the piezoelectric actuator according to the embodiment of the present disclosure;
FIG. 9A is a schematic cross-sectional view (1) illustrating an action of the piezoelectric actuator according to the embodiment of the present disclosure;
FIG. 9B is a schematic cross-sectional view (2) illustrating an action of the piezoelectric actuator according to the embodiment of the present disclosure;
FIG. 9C is a schematic cross-sectional view (3) illustrating an action of the piezoelectric actuator according to the embodiment of the present disclosure;
FIG. 10A is a schematic cross-sectional view (1) illustrating the gas detection main part according to the embodiment of the present disclosure;
FIG. 10B is a schematic cross-sectional view (2) illustrating the gas detection main part according to the embodiment of the present disclosure;
FIG. 10C is a schematic cross-sectional view (3) illustrating the gas detection main part according to the embodiment of the present disclosure;
FIG. 11 is a block diagram illustrating the communication of the gas detector according to the embodiment of the present disclosure; and
FIG. 12 is a block diagram illustrating the architecture of a cloud computing server according to the embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

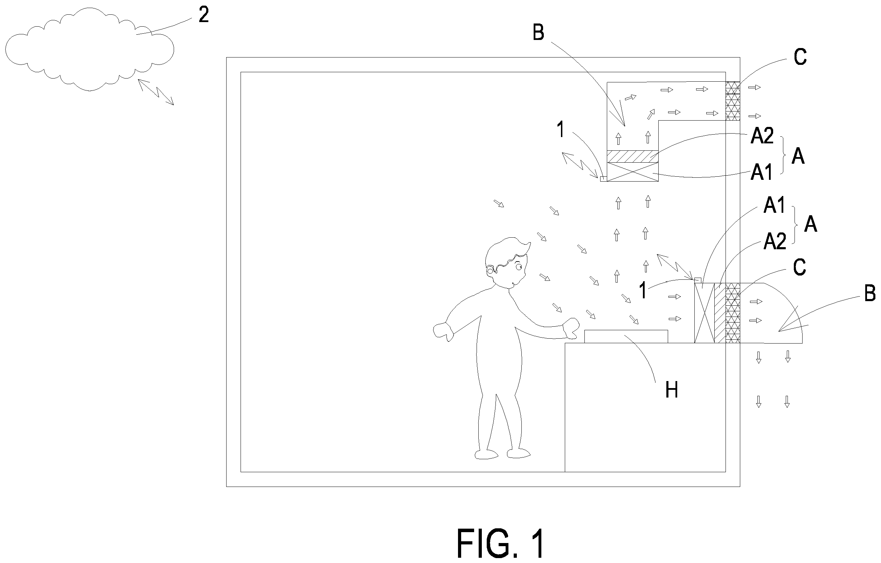

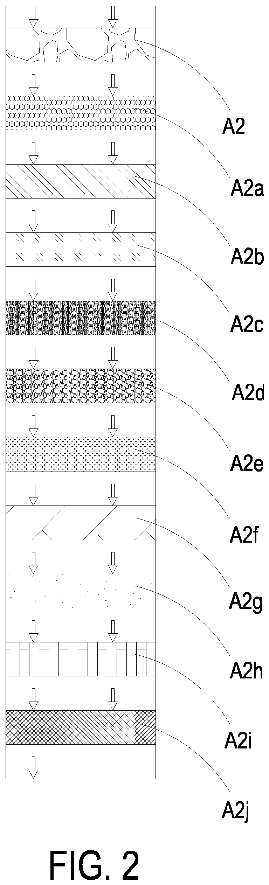

Please refer to FIG. 1 and FIG. 2. The present disclosure is related to an air pollution prevention system for a kitchen unit in an indoor field including a cooking device H, a plurality of negative pressure exhausting devices A, a plurality of gas detectors 1 and a cloud computing server 2.

The cooking device H mentioned above is disposed in the kitchen unit in the indoor field, and the air pollution is generated as the cooking device H is enabled to cook food. Notably, the air pollution includes at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus, or the combination thereof.

The plurality of negative pressure exhausting devices A mentioned above are disposed in front of and above the cooking device H. Each of the negative pressure exhausting devices A is connected to an exhausting channel B and includes at least one fan A1 and at least one filer element A2. The fan A1 is controlled to produce a negative pressure for guiding the air pollution generated by the cooking device H to enter the exhausting channel B and pass through the filter element A2 for filtration, so as to exhaust to the outdoor. Notably, in the embodiment, the negative pressure exhausting device A which is disposed in front of the cooking device H is a range hood for directly drawing out the air pollution so as to prevent the cook from smelling fumes, and the negative pressure exhausting device A which is disposed above the cooking device H is an exhaust fan for avoiding the air pollution from diffusing to other spaces, such as the living room, but not limited thereto.

The plurality of gas detectors 1 mentioned above are respectively disposed on the plurality of negative pressure exhausting devices A for detecting the air pollution and outputting air pollution information, and also for receiving a control command for controlling the respective negative pressure exhausting device A. Notably, the gas detector 1 is electrically connected to a driving circuit (not shown) of the fan A1 of the negative pressure exhausting device A, and after receiving the control command, the gas detector 1 enables the fan 21 and adjusts the air volume and operation time thereof. Moreover, a valve C is further disposed between the exhausting channel B and the outdoor, and the valve C is also controlled by the control command, which is received by the gas detector 1, to open at the same time.

The cloud computing server mentioned above 2 receives the air pollution information from the plurality of gas detectors 1, stores the air pollution information to an air pollution database, performs an intelligent computing to determine the concentration of air pollution, and issues the control command to the plurality of negative pressure exhausting devices A to control the enablement of the fans A1 thereof. Whereby, the air pollution generated in the kitchen unit is rapidly guided to pass through the filter element A2 for filtration and output to the outdoor. In the embodiment, when the cooking device H is enabled to cook, the transmission of the control command is enabled, and after the gas detectors 1 receive the control command, the negative exhausting devices A are enabled, so that the air pollution generated in the kitchen unit is guided to rapidly flow into the plurality of negative pressure exhausting devices A for exhausting to the outdoor, thereby achieving a gas state in the kitchen unit with a level of air pollution close to zero. Notably, the air pollution information mentioned above includes a detection value of fumes, VOC and/or polycyclic aromatic hydrocarbons, and when the detection vale is higher than a preset safe detection value, the cloud computing server 2 issues the control command to enable the plurality of negative pressure exhausting devices A and control the air volumes and operation times of the fans A1 based on the air pollution information, so as to rapidly guide the air pollution generated in the kitchen unit to flow into the plurality of negative pressure exhausting devices A for outputting to the outdoor, thereby achieving a gas state in the kitchen unit with a level of air pollution close to zero.

Please refer to FIG. 2. The filter element A2 mentioned above is a filter screen. Preferably but not exclusively, the filter screen is an ultra low particulate air (ULPA) filter screen or a high efficiency particulate air (HEPA) filter screen which is configured to absorb the chemical smoke, the bacteria, the dust particles and/or the pollen contained in the air pollution, so as to achieve the effects of filtration and purification. Moreover, the filter element A2 is combined with a material which provides physical or chemical sterilization effect. In the embodiment, the filter element A2 is coated with a decomposition layer for sterilizing in chemical means. Preferably but not exclusively, the decomposition layer includes an activated carbon layer A2a configured to remove organic and inorganic substances in the air pollution, and remove colored and odorous substances. Preferably but not exclusively, the decomposition layer includes a cleansing factor containing chlorine dioxide layer A2b configured to inhibit viruses, bacteria, fungi, influenza A, influenza B, enterovirus and norovirus in the air pollution, and the inhibition ratio can reach 99% and more, thereby reducing the cross-infection of viruses. Preferably but not exclusively, the decomposition layer includes an herbal protective layer A2c extracted from ginkgo and Japanese Rhus chinensis configured to resist allergy effectively and destroy a surface protein of influenza virus (such as H1N1 influenza virus) passing therethrough. Preferably but not exclusively, the decomposition layer includes a silver ion layer A2d configured to inhibit viruses, bacteria and fungi contained in the air pollution. Preferably but not exclusively, the decomposition layer includes a zeolite layer A2e configured to remove ammonia nitrogen, heavy metals, organic pollutants, Escherichia coli, phenol, chloroform and anionic surfactants. Furthermore, in some embodiments, the filter element A2 is combined with a light irradiation element to sterilize in chemical means. Preferably but not exclusively, the light irradiation element is a photo-catalyst unit including a photo catalyst A2f and an ultraviolet lamp A2g. When the photo catalyst A2f is irradiated by the ultraviolet lamp A2g, the light energy is converted into the electrical energy, thereby decomposing harmful substances and disinfects bacteria contained in the air pollution, so as to achieve the effects of filtration and sterilization. Preferably but not exclusively, the light irradiation element is a photo-plasma unit including a nanometer irradiation tube A2h. When the introduced air pollution is irradiated by the nanometer irradiation tube A2h, the oxygen molecules and water molecules contained in the air pollution are decomposed into high oxidizing photo-plasma, and an ion flow capable of destroying organic molecules is generated. In that, volatile formaldehyde, volatile toluene and volatile organic compounds (VOC) contained in the air pollution are decomposed into water and carbon dioxide, so as to achieve the effects of filtration and sterilization. In addition, in some embodiments, the filter element A2 is combined with a decomposition unit to sterilize in chemical means. Preferably but not exclusively, the decomposition unit is a negative ion unit A2i which makes the suspended particles in the air pollution to carry with positive charge and adhere to a dust collecting plate carry with negative charges, so as to achieve the effects of filtration and sterilization. Preferably but not exclusively, the decomposition unit is a plasma ion unit A2j. The oxygen molecules and water molecules contained in the air pollution are decomposed into positive hydrogen ions (H⁺) and negative oxygen ions (O²⁻) by the plasma ion. The substances attached with water around the ions are adhered on the surfaces of viruses and bacteria and converted into OH radicals with extremely strong oxidizing power, thereby removing hydrogen (H) from the protein on the surfaces of viruses and bacteria, and thus decomposing (oxidizing) the protein, so as to filter the introduced air pollution and achieve the effects of filtration and sterilization.

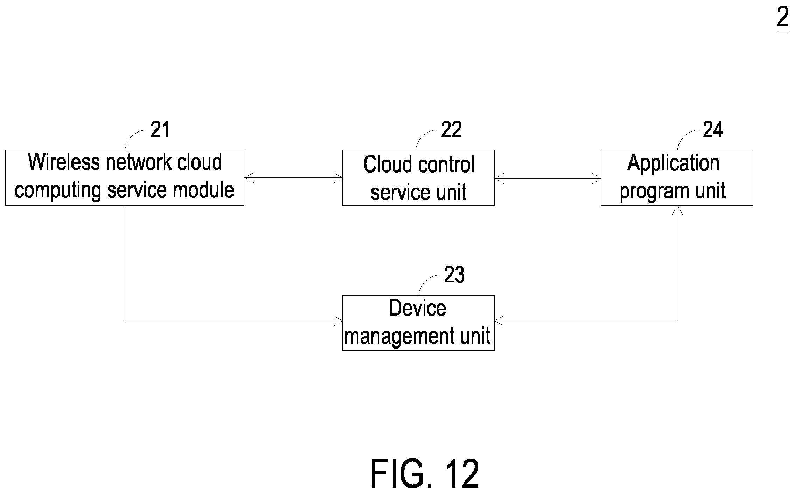

Please refer to FIG. 12. In the embodiment, the cloud computing server 2 includes a wireless network cloud computing service module 21, a cloud control service unit 22, a device management unit 23 and an application program unit 24. The wireless network cloud computing service module 21 receives the air pollution information of the kitchen unit in the indoor field, receives communication information of the fans 21, and issues the control command. Moreover, the wireless network cloud computing service module 21 receives the air pollution information from the kitchen unit in the indoor field and transmits thereof to the cloud control service unit 22 to store and form an air pollution database. The intelligence computing and comparison based on the air pollution database are implemented to determine the concentration of air pollution. Accordingly, the control command is transmitted to the wireless network cloud computing service module 21, and then transmitted through the wireless network cloud computing service module 21 to the fans A1 of the negative pressure exhausting devices A to control the enablement thereof, and at the same time, open the valve C. The device management unit 23 receives the communication information of the fans A1 through the wireless network cloud computing service module 21 to manage the user login and device binding, and the device management information can be provided to the application program unit 24 for system control and management. The application program unit 24 can also display and inform the air pollution information obtained from the cloud control service unit 22, informing the user the real-time status of air pollution removal through the mobile phone or the communication device. Moreover, the user can control the operation of the air pollution prevention system for kitchen unit in indoor field through the application program unit 24 of the mobile phone or the communication device.

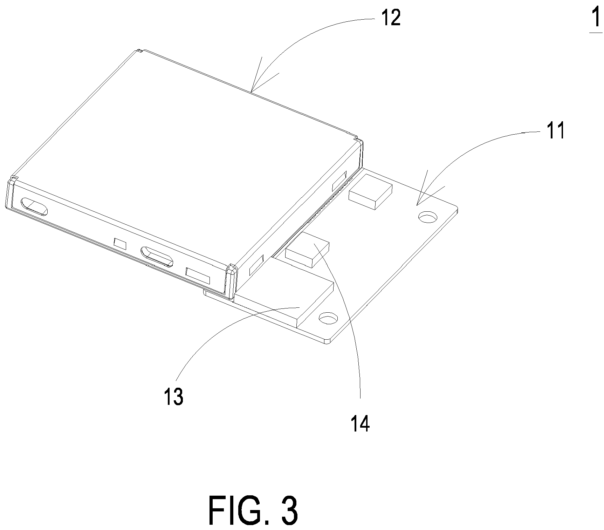

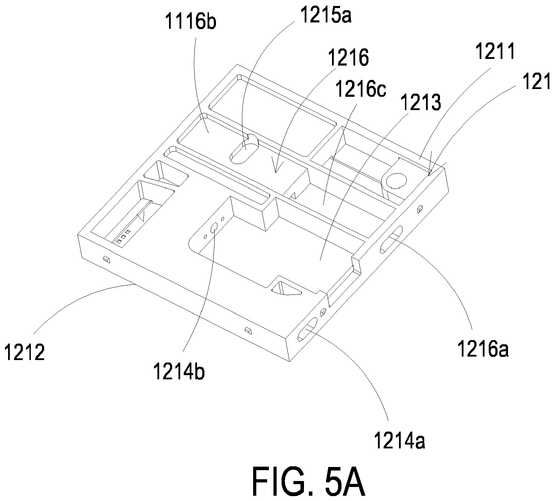

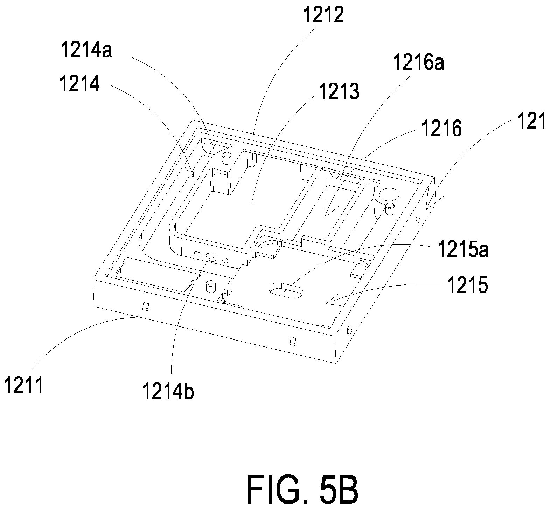

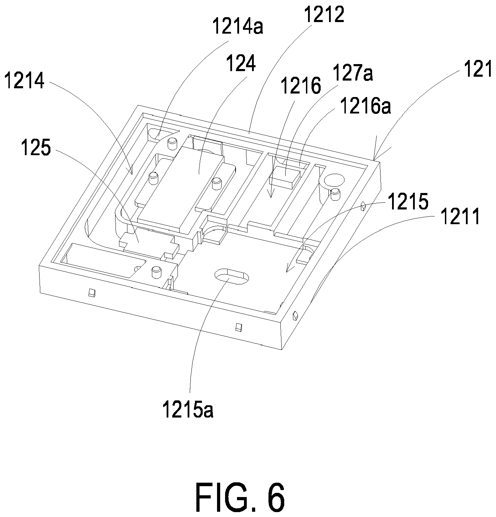

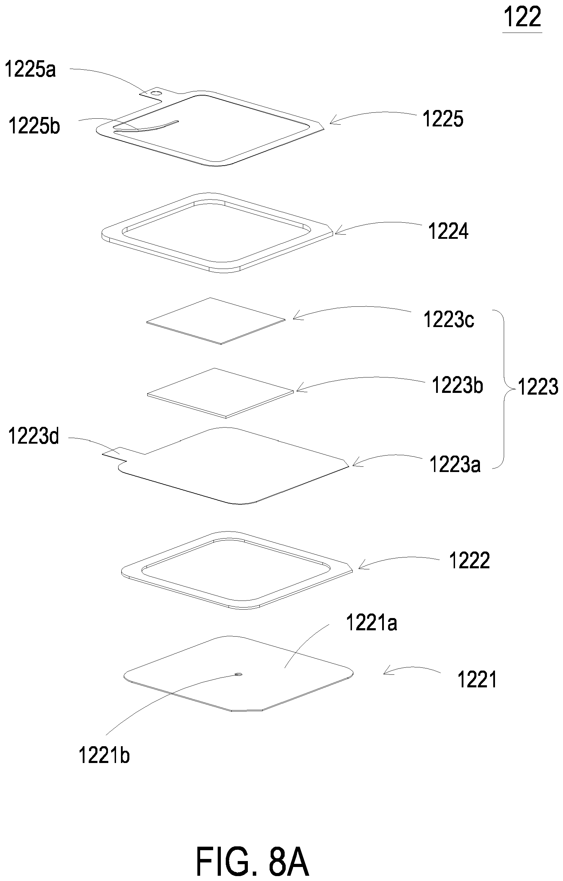

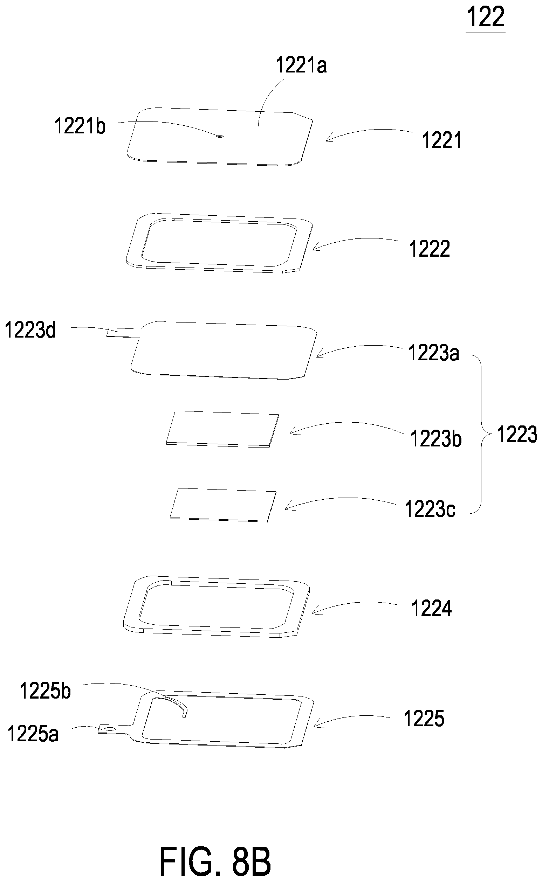

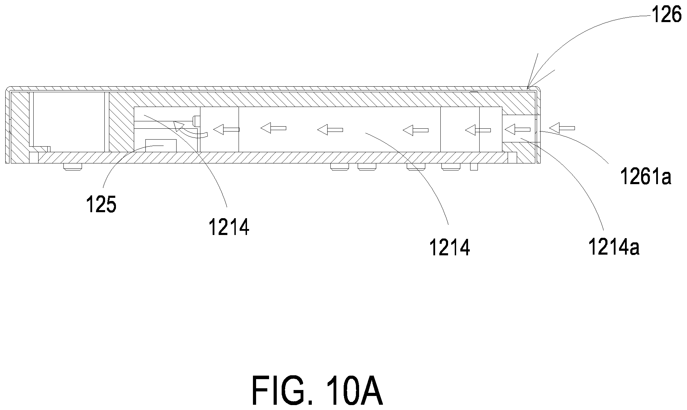

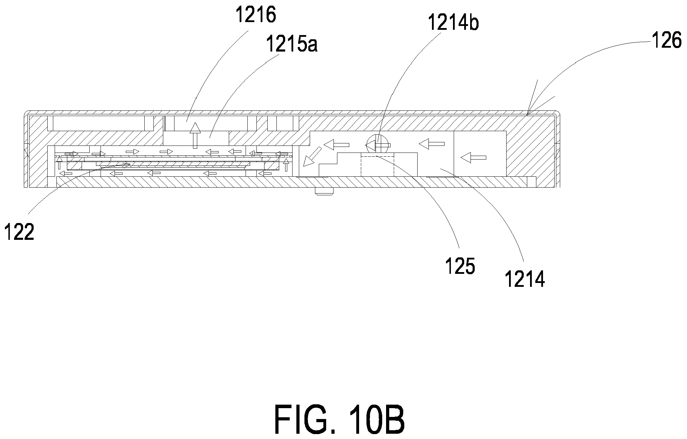

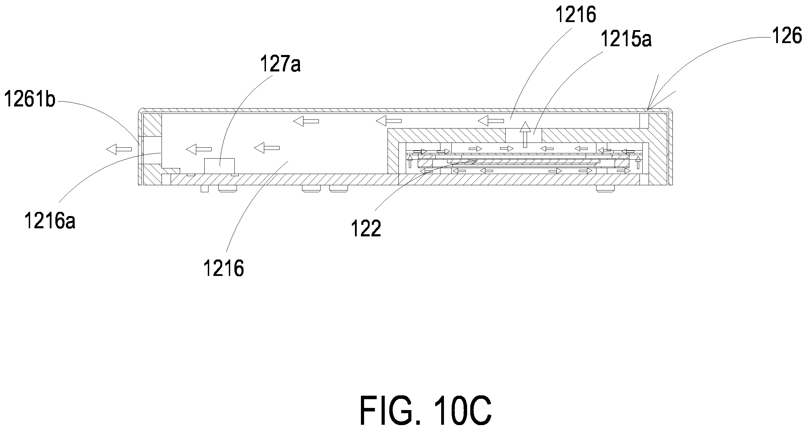

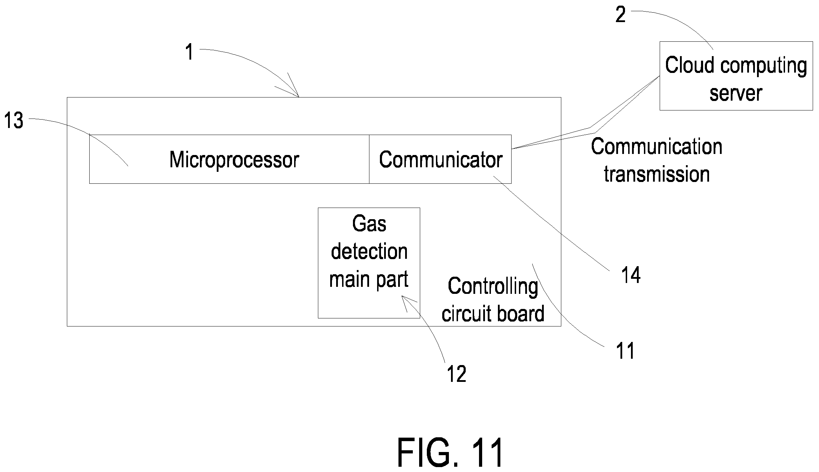

Please refer to FIG. 3A to FIG. 11. In the embodiment, the gas detector 1 includes a controlling circuit board 11, a gas detection main part 12, a microprocessor 13 and a communicator 14. The gas detection main part 12, the microprocessor 13 and the communicator 14 are integrally packaged on the controlling circuit board 11 and electrically connected to each other. The microprocessor 13 and the communicator 14 are mounted on the controlling circuit board 11. The microprocessor 13 controls the driving signal of the gas detection main part 12 for enabling the detection operation. In this way, the gas detection main part 12 detects the air pollution and outputs the air pollution information, and the microprocessor 13 receives, processes and provides the air pollution information to the communicator 14 for external communication transmission, so as to transmit to the cloud computing server 2.

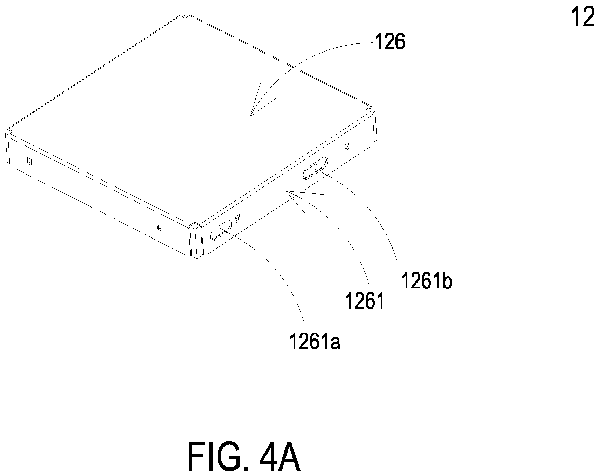

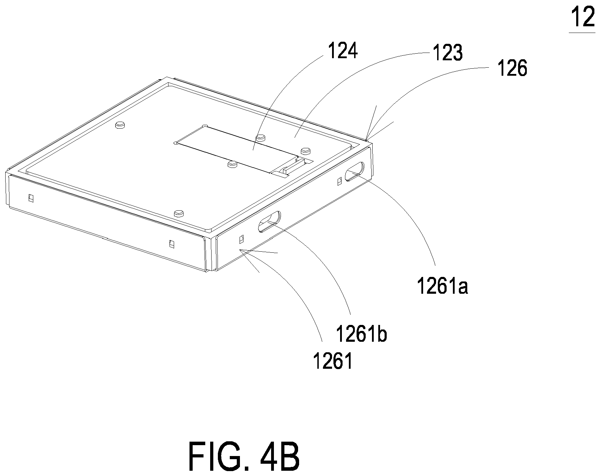

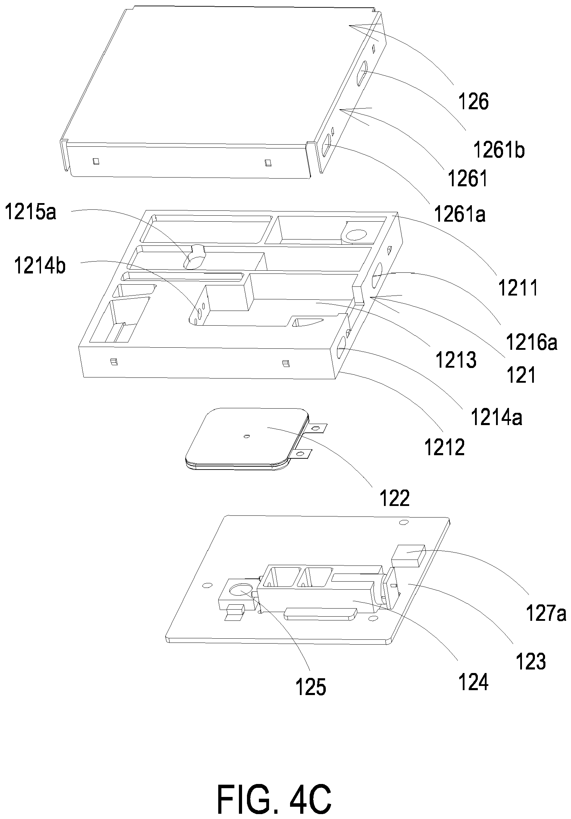

Please refer to FIG. 4A to FIG. 9A. The gas detection main part 12 includes a base 121, a piezoelectric actuator 122, a driving circuit board 123, a laser component 124, a particulate sensor 125, and an outer cover 126. In the embodiment, the base 121 includes a first surface 1211, a second surface 1212, a laser loading region 1213, a gas-inlet groove 1214, a gas-guiding-component loading region 1215 and a gas-outlet groove 1216. The first surface 1211 and the second surface 1212 are two surfaces opposite to each other. The laser loading region 1213 is hollowed out from the first surface 1211 toward the second surface 1212. The outer cover 126 covers the base 121 and includes a side plate 1261. The side plate 1261 has an inlet opening 1261a and an outlet opening 1261b. The gas-inlet groove 1214 is concavely formed from the second surface 1212 and disposed adjacent to the laser loading region 1213. The gas-inlet groove 1214 includes a gas-inlet 1214a and two lateral walls. The gas-inlet 1214a is in communication with an environment outside the base 121, and is spatially corresponding in position to an inlet opening 1261a of the outer cover 126. Two transparent windows 1214b are opened on the two lateral walls of the gas-inlet groove 1214 and are in communication with the laser loading region 1213. Therefore, the first surface 1211 of the base 121 is covered and attached by the outer cover 126, and the second surface 1212 is covered and attached by the driving circuit board 123, so that an inlet path is defined by the gas-inlet groove 1214.

In the embodiment, the gas-guiding-component loading region 1215 is concavely formed from the second surface 1212 and in communication with the gas-inlet groove 1214. A ventilation hole 1215a penetrates a bottom surface of the gas-guiding-component loading region 1215. The gas-guiding-component loading region 1215 includes four positioning protrusions 1215b disposed at four corners of the gas-guiding-component loading region 1215, respectively. In the embodiment, the gas-outlet groove 1216 includes a gas-outlet 1216a, and the gas-outlet 1216a is spatially corresponding to the outlet opening 1261b of the outer cover 126. The gas-outlet groove 1216 includes a first section 1216b and a second section 1216c. The first section 1216b is concavely formed out from the first surface 1211 in a region spatially corresponding to a vertical projection area of the gas-guiding-component loading region 1215. The second section 1216c is hollowed out from the first surface 1211 to the second surface 1212 in a region where the first surface 1211 is extended from the vertical projection area of the gas-guiding-component loading region 1215. The first section 1216b and the second section 1216c are connected to form a stepped structure. Moreover, the first section 1216b of the gas-outlet groove 1216 is in communication with the ventilation hole 1215a of the gas-guiding-component loading region 1215, and the second section 1216c of the gas-outlet groove 1216 is in communication with the gas-outlet 1216a. In that, when first surface 1211 of the base 121 is attached and covered by the outer cover 126 and the second surface 1212 of the base 121 is attached and covered by the driving circuit board 123, the gas-outlet groove 1216 and the driving circuit board 123 collaboratively define an outlet path.

In the embodiment, the laser component 124 and the particulate sensor 125 are disposed on and electrically connected to the driving circuit board 123 and located within the base 121. In order to clearly describe and illustrate the positions of the laser component 124 and the particulate sensor 125 in the base 121, the driving circuit board 123 is intentionally omitted. The laser component 124 is accommodated in the laser loading region 1213 of the base 121, and the particulate sensor 125 is accommodated in the gas-inlet groove 1214 of the base 121 and is aligned to the laser component 124. In addition, the laser component 124 is spatially corresponding to the transparent window 1214b, so that a light beam emitted by the laser component 124 passes through the transparent window 1214b and is irradiated into the gas-inlet groove 1214. A light beam path emitted from the laser component 124 passes through the transparent window 1214b and extends in an orthogonal direction perpendicular to the gas-inlet groove 1214. In the embodiment, a projecting light beam emitted from the laser component 124 passes through the transparent window 1214b and enters the gas-inlet groove 1214 to irradiate the suspended particles contained in the gas passing through the gas-inlet groove 1214. When the suspended particles contained in the gas are irradiated and generate scattered light spots, the scattered light spots are received and calculated by the particulate sensor 125, which is in an orthogonal direction perpendicular to the gas-inlet groove 1214, to obtain the gas detection information. Furthermore, a gas sensor 127a is positioned and disposed on the driving circuit board 123, electrically connected to the driving circuit board 123, and accommodated in the gas-outlet groove 1216, so as to detect the air pollution introduced therein. Preferably but not exclusively, in an embodiment, the gas sensor 127a includes a volatile-organic-compound sensor for detecting the information of carbon dioxide (CO₂) or volatile organic compounds (TVOC). Preferably but not exclusively, in an embodiment, the gas sensor 127a includes a formaldehyde sensor for detecting the information of formaldehyde (HCHO) gas. Preferably but not exclusively, in an embodiment, the gas sensor 127a includes a bacteria sensor for detecting the information of bacteria or fungi. Preferably but not exclusively, in an embodiment, the gas sensor 127a includes a virus sensor for detecting the information of virus in the gas.

In the embodiment, the piezoelectric actuator 122 is accommodated in the square-shaped gas-guiding-component loading region 1215 of the base 121. In addition, the gas-guiding-component loading region 1215 is in fluid communication with the gas-inlet groove 1214. When the piezoelectric actuator 122 is enabled, the gas in the gas-inlet groove 1214 is inhaled by the piezoelectric actuator 122, so that the gas flows into the piezoelectric actuator 122, and is transported into the gas-outlet groove 1216 through the ventilation hole 1215a of the gas-guiding-component loading region 1215. Moreover, the driving circuit board 123 covers the second surface 1212 of the base 121, and the laser component 124 is positioned and disposed on the driving circuit board 123, and is electrically connected to the driving circuit board 123. The particulate sensor 125 is also positioned and disposed on the driving circuit board 123 and electrically connected to the driving circuit board 123. In that, when the outer cover 126 covers the base 121, the inlet opening 1261a is spatially corresponding to the gas-inlet 1214a of the base 121, and the outlet opening 126lb is spatially corresponding to the gas-outlet 1216a of the base 121.

In the embodiment, the piezoelectric actuator 122 includes a gas-injection plate 1221, a chamber frame 1222, an actuator element 1223, an insulation frame 1224 and a conductive frame 1225. In the embodiment, the gas-injection plate 1221 is made by a flexible material and includes a suspension plate 1221a and a hollow aperture 1221b. The suspension plate 1221a is a sheet structure and is permitted to undergo a bending deformation. Preferably but not exclusively, the shape and the size of the suspension plate 1221a are corresponding to the inner edge of the gas-guiding-component loading region 1215, but not limited thereto. The hollow aperture 1221b passes through a center of the suspension plate 1221a, so as to allow the gas to flow therethrough. Preferably but not exclusively, in the embodiment, the shape of the suspension plate 1221a is selected from the group consisting of a square, a circle, an ellipse, a triangle and a polygon, but not limited thereto.

In the embodiment, the chamber frame 1222 is carried and stacked on the gas-injection plate 1221. In addition, the shape of the chamber frame 1222 is corresponding to the gas-injection plate 1221. The actuator element 1223 is carried and stacked on the chamber frame 1222 and collaboratively defines a resonance chamber 1226 with the chamber frame 1222 and the gas-injection plate 1221. The insulation frame 1224 is carried and stacked on the actuator element 1223 and the appearance of the insulation frame 1224 is similar to that of the chamber frame 1222. The conductive frame 1225 is carried and stacked on the insulation frame 1224, and the appearance of the conductive frame 1225 is similar to that of the insulation frame 1224. In addition, the conductive frame 1225 includes a conducting pin 1225a and a conducting electrode 1225b. The conducting pin 1225a is extended outwardly from an outer edge of the conductive frame 1225, and the conducting electrode 1225b is extended inwardly from an inner edge of the conductive frame 1225. Moreover, the actuator element 1223 further includes a piezoelectric carrying plate 1223a, an adjusting resonance plate 1223b and a piezoelectric plate 1223c. The piezoelectric carrying plate 1223a is carried and stacked on the chamber frame 1222. The adjusting resonance plate 1223b is carried and stacked on the piezoelectric carrying plate 1223a. The piezoelectric plate 1223c is carried and stacked on the adjusting resonance plate 1223b. The adjusting resonance plate 1223b and the piezoelectric plate 1223c are accommodated in the insulation frame 1224. The conducting electrode 1225b of the conductive frame 1225 is electrically connected to the piezoelectric plate 1223c. In the embodiment, the piezoelectric carrying plate 1223a and the adjusting resonance plate 1223b are made by a conductive material. The piezoelectric carrying plate 1223a includes a piezoelectric pin 1223d. The piezoelectric pin 1223d and the conducting pin 1225a are electrically connected to a driving circuit (not shown) of the driving circuit board 123, so as to receive a driving signal, such as a driving frequency and a driving voltage. Through this structure, a circuit is formed by the piezoelectric pin 1223d, the piezoelectric carrying plate 1223a, the adjusting resonance plate 1223b, the piezoelectric plate 1223c, the conducting electrode 1225b, the conductive frame 1225 and the conducting pin 1225a for transmitting the driving signal. Moreover, the insulation frame 1224 is insulated between the conductive frame 1225 and the actuator element 1223, so as to avoid the occurrence of a short circuit. Thereby, the driving signal is transmitted to the piezoelectric plate 1223c. After receiving the driving signal, the piezoelectric plate 1223c deforms due to the piezoelectric effect, and the piezoelectric carrying plate 1223a and the adjusting resonance plate 1223b are further driven to generate the bending deformation in the reciprocating manner.

Furthermore, in the embodiment, the adjusting resonance plate 1223b is located between the piezoelectric plate 1223c and the piezoelectric carrying plate 1223a and served as a cushion between the piezoelectric plate 1223c and the piezoelectric carrying plate 1223a. Thereby, the vibration frequency of the piezoelectric carrying plate 1223a is adjustable. Basically, the thickness of the adjusting resonance plate 1223b is greater than the thickness of the piezoelectric carrying plate 1223a, and the vibration frequency of the actuator element 1223 can be adjusted by adjusting the thickness of the adjusting resonance plate 1223b.

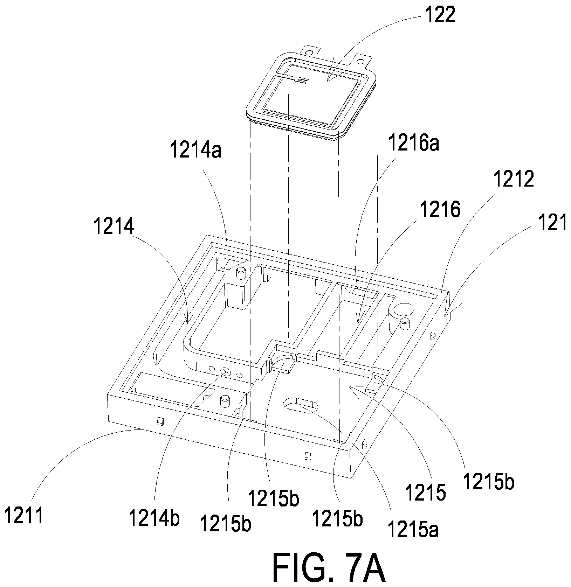

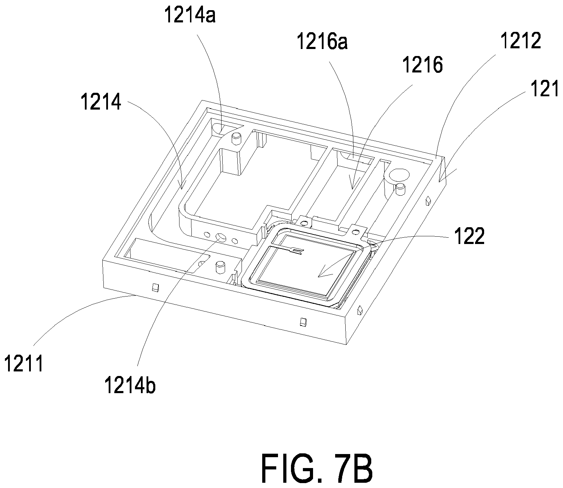

Please further refer to FIG. 7A, FIG. 7B, FIG.8A, FIG. 8B and FIG. 9A. In the embodiment, the gas-injection plate 1221, the chamber frame 1222, the actuator element 1223, the insulation frame 1224 and the conductive frame 1225 are stacked and positioned in the gas-guiding-component loading region 1215 sequentially, so that the piezoelectric actuator 122 is supported and positioned in the gas-guiding-component loading region 1215. A clearance 1221c is defined between the suspension plate 1221a and an inner edge of the gas-guiding-component loading region 1215 for gas flowing therethrough. In the embodiment, a flowing chamber 1227 is formed between the gas-injection plate 1221 and the bottom surface of the gas-guiding-component loading region 1215. The flowing chamber 1227 is in communication with the resonance chamber 1226 between the actuator element 1223, the chamber frame 1222 and the gas-injection plate 1221 through the hollow aperture 1221b of the gas-injection plate 1221. By controlling the vibration frequency of the gas in the resonance chamber 1226 to be close to the vibration frequency of the suspension plate 1221a, the Helmholtz resonance effect is generated between the resonance chamber 1226 and the suspension plate 1221a, so as to improve the efficiency of gas transportation. When the piezoelectric plate 1223c is moved away from the bottom surface of the gas-guiding-component loading region 1215, the suspension plate 1221a of the gas-injection plate 1221 is driven to move away from the bottom surface of the gas-guiding-component loading region 1215 by the piezoelectric plate 1223c. In that, the volume of the flowing chamber 1227 is expanded rapidly, the internal pressure of the flowing chamber 1227 is decreased to form a negative pressure, and the gas outside the piezoelectric actuator 122 is inhaled through the clearance 1221c and enters the resonance chamber 1226 through the hollow aperture 1221b. Consequently, the pressure in the resonance chamber 1226 is increased to generate a pressure gradient. When the suspension plate 1221a of the gas-injection plate 1221 is driven by the piezoelectric plate 1223c to move toward the bottom surface of the gas-guiding-component loading region 1215, the gas in the resonance chamber 1226 is discharged out rapidly through the hollow aperture 1221b, and the gas in the flowing chamber 1227 is compressed, thereby the converged gas is quickly and massively ejected out of the flowing chamber 1227 under the condition close to an ideal gas state of the Benulli's law, and transported to the ventilation hole 1215a of the gas-guiding-component loading region 1215.

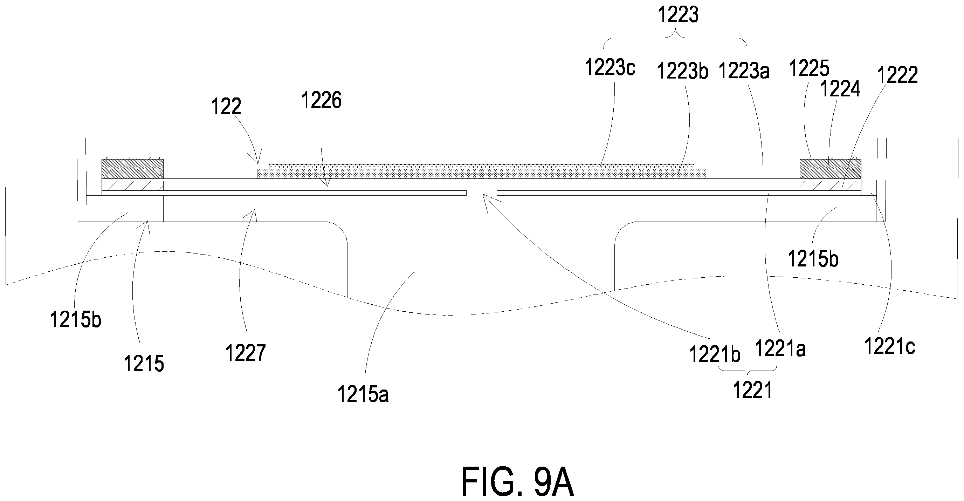

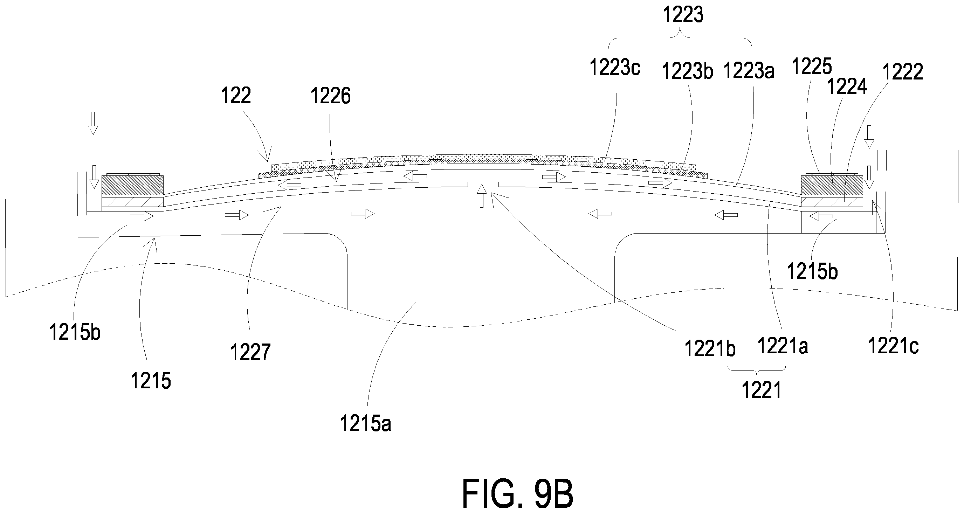

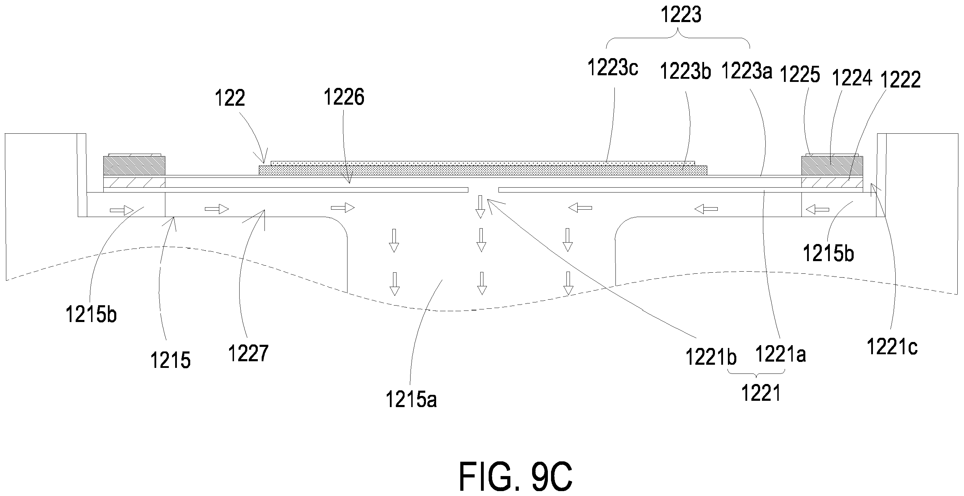

By repeating the above operation steps shown in FIG. 9B and FIG. 9C, the piezoelectric plate 1223c is driven to generate the bending deformation in a reciprocating manner. According to the principle of inertia, since the gas pressure inside the resonance chamber 1226 is lower than the equilibrium gas pressure after the converged gas is ejected out, the gas is introduced into the resonance chamber 1226 again. Moreover, the vibration frequency of the gas in the resonance chamber 1226 is controlled to be close to the vibration frequency of the piezoelectric plate 1223c, so as to generate the Helmholtz resonance effect to achieve the gas transportation at high speed and in large quantities. The gas is inhaled through the inlet opening 1261a on the outer cover 126, flows into the gas-inlet groove 1214 of the base 121 through the gas-inlet 1214a, and is transported to the position of the particulate sensor 125. The piezoelectric actuator 122 is enabled continuously to inhale the gas into the inlet path, and facilitate the gas outside the gas detection module to be introduced rapidly, flow stably, and transported above the particulate sensor 125. At this time, a projecting light beam emitted from the laser component 124 passes through the transparent window 1214b to irritate the suspended particles contained in the gas flowing above the particulate sensor 125 in the gas-inlet groove 1214. When the suspended particles contained in the gas are irradiated and generate scattered light spots, the scattered light spots are received and calculated by the particulate sensor 125 for obtaining related information about the sizes and the concentration of the suspended particles contained in the gas. Moreover, the gas above the particulate sensor 125 is continuously driven and transported by the piezoelectric actuator 122, flows into the ventilation hole 1215a of the gas-guiding-component loading region 1215, and is transported to the gas-outlet groove 1216. At last, after the gas flows into the gas outlet groove 1216, the gas is continuously transported into the gas-outlet groove 1216 by the piezoelectric actuator 122, and thus the gas in the gas-outlet groove 1216 is pushed to discharge through the gas-outlet 1216a and the outlet opening 1261b.

The gas detector 1 of the present disclosure not only can detect the particulate matters in the gas, but also can detect the gas characteristics of the introduced gas, for example, to determine whether the gas is formaldehyde, ammonia, carbon monoxide, carbon dioxide, oxygen, ozone, or the like. Therefore, the gas detector 1 of the present disclosure further includes a gas sensor 127a positioned and disposed on the driving circuit board 123, electrically connected to the driving circuit board 123, and accommodated in the gas-outlet groove 1216, so as to detect the gas characteristics of the introduced gas.

In summary, the present disclosure provides the air pollution prevention system for the kitchen in the indoor field. For solving the problem that the air pollution from the kitchen unit in the indoor field might be generated and moved anytime, the plurality of negative pressure exhausting devices in the present disclosure are disposed above and in front of the cooking device, and the plurality of gas detectors are disposed on the plurality of negative pressure exhausting devices for detecting the air pollution and outputting air pollution information and for receiving the control command to enable the plurality of negative pressure exhausting devices. Moreover, the cloud computing server receives the air pollution information detected by the plurality of gas detectors, stores the air pollution information to the air pollution database, performs the intelligence computing to determine the concentration of air pollution, and issues the control command to the plurality of negative pressure exhausting devices for enabling the fans thereof and also adjusting the air volume and operation time of the fans, so that the air pollution from the kitchen unit is rapidly guided to pass through the filter element for filtration and exhaust to the outdoor. Whereby, the air pollution is directly drawn out for preventing the cook from smelling fumes, and avoiding the diffusion of air pollution to other spaces, such as the living room. Hence, the present disclosure is extremely industrially applicable.

## Claims

1. An air pollution prevention system for a kitchen unit in an indoor field, **characterized by** comprising:
a cooking device (H), wherein an air pollution is generated when the cooking device (H) is enabled to cook;
a plurality of negative pressure exhausting devices (A) disposed above and in front of the cooking device (H) and respectively connected to an exhausting channel (B), wherein each of the plurality of negative pressure exhausting devices (A) comprises at least one fan (A1) and at least one filter element (A2), and the at least one fan (A1) is controlled to generate a negative pressure for guiding the air pollution to flow into the respective exhausting channel (B) and pass through the at least one filter element (A2) for filtration and exhausting to an outdoor;
a plurality of gas detectors (1) disposed on the plurality of negative pressure exhausting devices (A) for detecting the air pollution and outputting air pollution information, receiving a control command to enable the plurality of negative pressure exhausting devices (A); and
a cloud computing server (2) receiving the air pollution information detected by the plurality of negative pressure exhausting devices (A), storing the air pollution information to an air pollution database, performing an intelligent computing for determining a concentration of the air pollution, and issuing the control command to the plurality of negative pressure exhausting devices (A) to enable the fans (A1) of the plurality of negative pressure exhausting devices (A), thereby rapidly guiding the air pollution from the kitchen unit to pass through the filter elements (A2) for filtration and purification and to exhaust to the outdoor,
wherein a transmission of the control command is enabled when the cooking device (H) is enabled to cook, after the plurality of gas detectors (1) receive the control command, the plurality of negative pressure exhausting devices (A) are enabled, thereby the air pollution from the kitchen unit is guided to rapidly flow into the plurality of negative pressure exhausting devices (A) for exhausting to the outdoor, achieving a gas state in the kitchen unit with a level of air pollution close to zero.

2. The air pollution prevention system for the kitchen unit in the indoor field according to claim 1, wherein the air pollution is at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus, or the combination thereof.

3. The air pollution prevention system for the kitchen unit in the indoor field according to claim 1, wherein the air pollution information comprises a detection value of at least one selected from the group consisting of fumes, VOC and polycyclic aromatic hydrocarbons, and wherein when the detection vale is higher than a preset safe detection value, the cloud computing server (2) issues the control command to enable the plurality of negative pressure exhausting devices (A) and adjust air volumes and operation times of the fans (A1) based on the air pollution information, so as to rapidly guide the air pollution generated in the kitchen unit to flow into the plurality of negative pressure exhausting devices (A) for exhausting to the outdoor, thereby achieving a gas state in the kitchen unit with a level of air pollution close to zero.

4. The air pollution prevention system for the kitchen unit in the indoor field according to claim 1, wherein the plurality of gas detectors (1) are disposed on the plurality of negative pressure exhausting devices (A) and electrically connected to driving circuits of the fans (A1) of the plurality of negative pressure exhausting devices (A) for adjusting air volumes and operation times of the fans (A1) after receiving the control command, a valve (C) is disposed between the respective exhausting channel (B) and the outdoor, and the valve (C) is simultaneously controlled by the control command which is received by the respective gas detector (1) for enabling the respective fan (A1).

5. The air pollution prevention system for the kitchen unit in the indoor field according to claim 1, wherein each of the plurality of gas detectors (1) comprises a controlling circuit board (11), a gas detection main part (12), a microprocessor (13) and a communicator (14); wherein the gas detection main part (12), the microprocessor (13) and the communicator (14) are integrally packaged on the controlling circuit board (11) and electrically connected to the controlling circuit board (11), the microprocessor (13) controls a detection operation of the gas detection main part (12), the gas detection main part (12) detects the air pollution and outputs the air pollution information, and the microprocessor (13) receives, processes and provides the air pollution information to the communicator (14) for external communication transmission.

6. The air pollution prevention system for the kitchen unit in the indoor field according to claim 5, wherein the gas detection main part (12) comprises:
a base (121) comprising:
a first surface (1211);
a second surface (1212) opposite to the first surface (1211);
a laser loading region (1213) hollowed out from the first surface (1211) to the second surface (1212)
a gas-inlet groove (1214) concavely formed from the second surface (1212) and disposed adjacent to the laser loading region (1213), wherein the gas-inlet groove (1214) comprises a gas-inlet (1214a) and two lateral walls, and a transparent window (1214b) is respectively opened on the two lateral walls and is in communication with the laser loading region (1213);
a gas-guiding-component loading region (1215) concavely formed from the second surface (1212) and in communication with the gas-inlet groove (1214), wherein a ventilation hole (1215a) penetrates a bottom surface of the gas-guiding-component loading region (1215); and
a gas-outlet groove (1216) concavely formed from the first surface (1211), spatially corresponding to the bottom surface of the gas-guiding-component loading region (1215), and hollowed out from the first surface (1211) to the second surface (1212) in a region where the first surface (1211) is not aligned with the gas-guiding-component loading region (1215), wherein the gas-outlet groove (1216) is in communication with the ventilation hole (1215a), and a gas-outlet (1216a) is disposed in the gas-outlet groove (1216);
a piezoelectric actuator (122) accommodated in the gas-guiding-component loading region (1215);
a driving circuit board (123) covering and attached to the second surface (1212) of the base (121);
a laser component (124) positioned and disposed on the driving circuit board (123), electrically connected to the driving circuit board (123), and accommodated in the laser loading region (1213), wherein a light beam path emitted from the laser component (124) passes through the transparent window (1214b) and extends in a direction perpendicular to the gas-inlet groove (1214), thereby forming an orthogonal direction with the gas-inlet groove (1214);
a particulate sensor (125) positioned and disposed on the driving circuit board (123), electrically connected to the driving circuit board (123), and disposed at an orthogonal position where the gas-inlet groove (1214) intersects the light beam path of the laser component (124) in the orthogonal direction, so that suspended particles contained in the air pollution passing through the gas-inlet groove (1214) and irradiated by a projecting light beam emitted from the laser component (124) are detected;
a gas sensor (127a) positioned and disposed on the driving circuit board (123), electrically connected to the driving circuit board (123), and accommodated in the gas-outlet groove (1216), so as to detect the air pollution introduced into the gas-outlet groove(1216); and
an outer cover (126) covering the base (121) and comprising a side plate (1261), wherein the side plate (1261) has an inlet opening (1261a) and an outlet opening (1261b), the inlet opening (1261a) is spatially corresponding to the gas-inlet (1214a) of the base (121), and the outlet opening (1261b) is spatially corresponding to the gas-outlet (1216a) of the base (121);
wherein the outer cover (126) covers the base (121), and the driving circuit board (123) covers the second surface (1212), thereby an inlet path is defined by the gas-inlet groove (1214), and an outlet path is defined by the gas-outlet groove (1216), so that the air pollution is inhaled from the environment outside the base (121) by the piezoelectric actuator (122), transported into the inlet path defined by the gas-inlet groove (1214) through the inlet opening (1261a), and passes through the particulate sensor (125) to detect the particle concentration of the suspended particles contained in the air pollution, and the air pollution transported through the piezoelectric actuator (122) is transported out of the outlet path defined by the gas-outlet groove (1216) through the ventilation hole (1215a), passes through the gas sensor (127a) for detecting, and then pushed to discharge through the gas-outlet (1216a) of the base (121) and the outlet opening (1261b).

7. The air pollution prevention system for the kitchen unit in the indoor field according to claim 6, wherein the particulate sensor (125) is used for detecting information of the suspended particulate, and the gas sensor (127a) comprises a volatile-organic-compound sensor for detecting information of at least one selected from the group consisting of carbon dioxide, polycyclic aromatic hydrocarbon, and total volatile organic compounds.

8. The air pollution prevention system for the kitchen unit in the indoor field according to claim 6, wherein the gas sensor (127a) comprises one selected from the group consisting of a formaldehyde sensor, a bacteria sensor, a virus sensor, or the combination thereof, for respectively detecting information of formaldehyde, information of bacteria or fungi, and information of virus.

9. The air pollution prevention system for the kitchen unit in the indoor field according to claim 1, wherein the filter element (A2) is a high efficiency particulate air (HEPA) filter screen which provides purification effect through physical blocking and absorption.

10. The air pollution prevention system for the kitchen unit in the indoor field according to claim 9, wherein the HEPA filter screen is combined with a decomposition layer coated thereon to purify the air pollution in chemical means.

11. The air pollution prevention system for the kitchen unit in the indoor field according to claim 10, wherein the decomposition layer comprises at least one selected from the group consisting of an activated carbon layer (A2a), a cleansing factor containing chlorine dioxide layer (A2b), an herbal protective layer (A2c) extracted from ginkgo and Japanese rhus chinensis, or the combination thereof.

12. The air pollution prevention system for the kitchen unit in the indoor field according to claim 10, wherein the decomposition layer comprises at least one selected from the group consisting of a silver ion layer (A2d), a zeolite layer (A2e), or the combination thereof.

13. The air pollution prevention system for the kitchen unit in the indoor field according to claim 1, wherein the filter element (A2) is combined with a light irradiation element to purify the air pollution in chemical means, and the light irradiation element is at least one selected from the group consisting of a photo-catalyst unit comprising a photo catalyst (A2f) and an ultraviolet lamp (A2g), a photo-plasma unit comprising a nanometer irradiation tube (A2g), or the combination thereof.

14. The air pollution prevention system for the kitchen unit in the indoor field according to claim 1, wherein the filter element (A2) is combined with a decomposition unit to purify the air pollution in chemical means, and the decomposition unit is at least one selected from the group consisting of a negative ion unit (A2i), a plasma ion unit (A2j), or the combination thereof.

15. The air pollution prevention system for the kitchen unit in the indoor field according to claim 1, wherein the cloud computing server (2) comprises a wireless network cloud computing service module (21), a cloud control service unit (22), a device management unit (23) and an application program unit (24), and the plurality of negative pressure exhausting devices (A) comprise at least one of a range hood, an exhaust fan, or the combination thereof.
